# EUROPÄISCHE PATENTSCHRIFT

(11) **EP 1 970 030 B1**
(45) Veröffentlichungstag und Bekanntmachung des Hinweises auf die Patenterteilung: **29.04.2009**
(21) Anmeldenummer: 08004209.6
(22) Anmeldetag: 07.03.2008
(51) Int. Cl.: A61F 2/38

(54) **Gelenk**
Joint
Articulation

(30) Priorität: 15.03.2007 DE 102007013121
(43) Veröffentlichungstag der Anmeldung: 17.09.2008
(73) Patentinhaber: Aequos Endoprothetik Gmbh, 82166 Gräfelfing (DE)
(72) Erfinder: Nägerl, Hans, Prof. Dr., 37130 Gleichen (DE)
(74) Vertreter: Scheffler, Jörg

(56) Entgegenhaltungen:
- EP-A- 0 734 700
- DE-A1- 19 521 597
- DE-C1- 10 231 538
- US-A- 4 309 778
- US-A- 5 133 758

## Beschreibung

Die Erfindung betrifft ein künstliches Gelenk als Endoprothese für ein menschliches Gelenk, bestehend aus mindest zwei Gelenkteilen, wobei jedes Gelenkteil zwei miteinander in Wirkverbindung stehende, jeweils dem ersten Gelenkteil einerseits und dem zweiten Gelenkteil andererseits zugeordnete Funktionsflächen aufweist, wobei die beiden Funktionsflächen jedes Gelenkteils sphäroidal geformt und in der proximal-distalen Zuordnung konvex-konkav, konkav-konvex oder konvex-konvex geformt sind, und die Funktionsflächen um jeweils eine Schwenkachse schwenkbeweglich sind. I-nsbesondere betrifft die Erfindung eine solche Endoprothese für das menschliche Kniegelenk, welches jeweils dem Femur einerseits und der Tibia andererseits zugeordnete Funktionsflächen aufweist, wobei die beiden Funktionsflächen jedes Gelenkteils sphäroidal geformt und in der femur-tibialen Zuordnung konvexkonkav, konkav-konvex oder konvex-konvex geformt sind.

Ein gattungsgemäßes Gelenk ist beispielsweise durch die EP 07 34 700 A2 bekannt, bei dem die Gelenkgeometrie der Funktionsflächen zueinander in jeder der beiden Ebenen durch eine Gelenkkette mit zwei Gelenkachsen bestimmt ist, die durch die Rotationszentren der Funktionsflächen mit den Radien der jeweils zugehörigen Schnittkonturen verlaufen, wobei eine femurseitige, also gelenkkopfseitige Verbindung der Mittelpunkte der Gelenkköpfe einem Gestell und eine tibiaseitige, also gelenkpfannenseitige Verbindung der Mittelpunkte der Gelenkpfannen einer Koppel einer die vier Achsen aufweisenden Viergelenkkette entsprechen.

Durch die DE 196 46 891 A1 ist ein künstliches Gelenk, insbesondere eine Endoprothese zum Ersatz natürlicher Gelenke, bestehend aus mindestens zwei künstlichen Gelenkteilen mit gekrümmten Artikulationsflächen, bekannt, wobei auf jeder der Artikulationsflächen eine gekrümmte Kontaktlinie ausgebildet ist. Die gekrümmte Kontaktlinie einer der Artikulationsflächen ist ein Teil einer elliptischen Schnittkontur eines ersten Zylinders oder Kegels mit dem Zylinderradius bzw. dem Kegelwinkel. Die andere Kontaktlinie ergibt sich als Gegenspur eines zweiten auf dem ersten Zylinder bzw. ersten Kegel abrollenden oder gleitenden zweiten Zylinders bzw. zweiten Kegels mit dem Zylinderradius bzw. mit dem Kegelwinkel. Die Artikulationstflächen weisen aus einer Vielzahl von geraden Berührungslinien gebildete Regelflächen auf. Diese Regelflächen schließen sich gegenseitig an die Kontaktlinien einander gegenüberliegend an und die Berührungslinien sind jeweils die Verbindungslinien zwischen einem momentanen Kontaktpunkt der Kontaktlinien und einem momentan gemeinsamen Punkt bzw. dem Momentanpol der jeweiligen Bewegungssysteme in einer Referenzebene bzw. einer Referenzkugel im bewegten/unbewegten System.

Auch die DE 195 21 597 A1 betrifft ein künstliches Gelenk, insbesondere eine Endoprothese zum Ersatz natürlicher Gelenke, bestehend aus mindestens zwei künstlichen Gelenkteilen mit gekrümmten Artikulationsflächen, wobei auf jeder der Artikulationsflächen eine kreisbogenförmige Kontaktlinie ausgebildet ist, die jeweils ein Teilabschnitt eines in einer Ebene liegenden Kontaktkreises mit einem Mittelpunkt ist. Die Artikulationsflächen sind derart als Paar zueinander angeordnet, dass die Kontaktlinien aufeinander abrollen können sowie senkrecht zu der Ebene der Kontaktkreise durch deren Mittelpunkt verlaufende Achsen sich in einem Schnittpunkt schneiden. Einseitig an die Kontaktlinien sind aus einer Vielzahl von geraden Berührungslinien gebildete Regelflächen angeformt, wobei die Berührungslinien auf momentanen Verbindungslinien der während der Abrollbewegung auftretenden momentanen Kontaktpunkte mit den momentanen Schnittpunkten liegen, welche sich durch eine Schwenkbewegung der Kontaktlinien mit einer Winkelgeschwindigkeit um eine gemeinsame Tangente der Kontaktlinien durch die momentanen Kontaktpunkte ergeben.

Die EP 600 806 A1 offenbart auch bereits eine dreiteilige Knieprothese mit einem femoralen und einem tibialen Implantat, wobei das femorale Implantat eine Einkerbung aufweist, durch die zwei auseinander laufende und durch eine Trochlea verbundene Kundylenauflageschuhe umschrieben werden, wobei das femorale Implantat eine Form besitzt, die sich aus der Kombination der folgenden Merkmale ergibt, dass nämlich der innere und der äußere Kondylenschuh in sagittaler Ebene unterschiedliche Krümmungsradien haben, die Auflage- und Gleitflächen des inneren und äußeren Kondylenschuhs in frontaler Ebene Unterschiede in Breite und Querschnitt aufweisen, der innere und äußere Kondylenschuh im hinteren Teil unterschiedliche Rollamplituden besitzen, die Trochlea in frontaler und sagittaler Ebene eine Fläche mit dem Querschnitt eines geometrischen Torus begrenzt, wobei sie auf der Außenseite in anatomischer Weise angehoben ist, die Spanne der Auflage- und Gleitflächen der Kondylenschuhe im Vergleich zu den anatomischen Verhältnissen erhöht ist und die Kondylenschuhe abgeflacht sind, um die Druckverteilung zu begünstigen.

Aus der deutschen Patentanmeldung DE 39 08 958 A1 ist bereits ein zum Ersatz menschlicher Gelenke bestimmtes künstliches Gelenk bekannt, welches aus zwei Gelenkteilen mit beweglichen Funktionsflächen besteht Die Krümmungsverhältnisse der eine kreisförmige Schnittkontur aufweisenden Funktionsflächen sind zueinander konvex-konvex, konvex-konkav oder konkav-konkav und die Gelenkgeometrie ist durch eine Gelenkkette mit zwei Gelenkachsen (dimere Gelenkkette) bestimmt, die durch die Rotationszentren der Funktionsflächen verlaufen. Hierbei sind die Gelenkflächen kugelförmig ausgebildet, so dass eine Gelenkbewegung mit fünf Freiheitsgraden möglich ist.

Der Erfindung liegt die Aufgabe zugrunde, ein künstliches Gelenk als Endoprothese für ein menschliches Gelenk zu schaffen, dessen Bewegungsverhalten für den Patienten spürbar verbessert ist.

Diese Aufgabe wird erfindungsgemäß mit einem Gelenk gemäß den Merkmalen des Anspruchs 1 gelöst. Die weitere Ausgestaltung der Erfindung ist den Unteransprüchen zu entnehmen.

Erfindungsgemäß ist also ein künstliches Gelenk für das menschliche Kniegelenk vorgesehen, bei dem die beiden Schwenkachsen der beiden Funktionsflächen jedes Gelenkteils zueinander geneigt sind. Hierdurch wird mit überraschend einfachen Mitteln erreicht, dass sich bei der Beugung des Kniegelenkes eine Bewegungsbahn ergibt, die nicht etwa in der saggitalen Ebene verläuft, sondern die einer räumlichen Bewegungsbahn entspricht. Mit anderen Worten führt die Neigung der Schwenkachsen zu einer aus der Hauptfunktionsebene lateral herausführenden Bahn, so dass der Fuß beim Gehen bezogen auf die Ebene der Bodenfläche entlang einer lateral bogenförmigen Bewegungsbahn geführt wird. Die Lage der momentanen Schraub- bzw. Drehachse ist dabei abhängig vom Flexionswinkel des Kniegelenks. Ein Mehrgelenk, das durch die beiden Schwenkachsen der tibialen Funktionsflächen und die beiden Schwenkachsen der korrespondierenden femoralen Funktionsflächen gebildet ist, gibt diese Lagen der momentanen Schraub- bzw. Drehachse in Abhängigkeit vom Flexionswinkel vor.

Die beiden Schwenkachsen der Funktionsflächen eines Gelenkteils können jeweils einen Schnittpunkt aufweisen. Eine besonders vorteilhafte Ausführungsform der vorliegenden Erfindung wird auch dadurch erreicht, dass die vier Schwenkachsen der beiden Funktionsflächen einander in einem gemeinsamen Schnittpunkt schneiden. Hierdurch wird eine zuverlässige, durch den Flexionswinkel bestimmte Abrollbewegung der beiden Funktionsflächen ohne eine gegenseitige Beeinträchtigung erreicht und so insbesondere ein zwangsweises Gleiten eines der beiden Funktionsflächenpaare oder gar eine Unterbrechung des Kontaktes der Funktionsflächen eines Gelenkteils vermieden. Die in einem vorbestimmten Flexionsbereich des Kniegelenkes gewünschte Gleitbewegung der Funktionsflächen kann selbstverständlich uneingeschränkt eingestellt werden.

Als besonders praxisnah erweist es sich hingegen auch, wenn der gemeinsame Schnittpunkt der Schwenkachsen der Funktionsflächen der beiden Gelenkteile auf der medialen Seite liegt, so dass sich eine Bewegungsbahn beispielsweise der Tibia entlang einer in lateraler Richtung konvex geformten Bewegungsbahn ergibt. Vereinfacht ausgedrückt wird der Flexionsbewegung beim Gehen oder Laufen eine Pendelbewegung des Unterschenkels des Patienten um sein Standbein herum überlagert, wobei der so erzielte Bewegungsablauf von dem Patienten als wesentlich angenehmer empfunden wird. Der gemeinsame Schnittpunkt der Schwenkachsen der Funktionsflächen der beiden Gelenkteile kann beispielsweise bei einer Anwendung als künstliches Daumengelenk radial angeordnet sein.

Eine andere ebenfalls besonders zweckmäßige Ausgestaltung des erfindungsgemäßen Gelenks wird dadurch erreicht, dass die Funktionsflächen in Bezug auf ihre jeweilige Schwenkachse als ein Rotationskörper ausgeführt sind. Beispielsweise kann der rotationssymmetrische Körper zu diesem Zweck spindel- oder tonnenförmig ausgeführt sein oder eine in Längserstreckung sphäroidale, beispielsweise elliptische oder toroide, Querschnittsform aufweisen.

Demgegenüber wird eine ebenfalls besonders sinnvolle Abwandlung der vorliegenden Erfindung dadurch erreicht, dass die Funktionsflächen abschnittsweise kegelstumpfförmig ausgeführt sind, um auf diese Weise einen Linienkontakt der Funktionsflächen eines Gelenkteils parallel zu der Schwenkachse zu ermöglichen. Beispielsweise könnte dieser Linienkontakt auf bestimmte Flexionswinkel des Kniegelenks beschränkt sein, bei denen besonders hohe Lastzustände auftreten.

Weiterhin erweist es sich als besonders praxisgerecht, wenn die Funktionsflächen in Abhängigkeit des Flexionswinkels des Gelenkes entweder aufeinander abrollen oder gleiten oder aber das Verhältnis des Abrollens und Gleitens in Abhängigkeit des Flexionswinkels variiert. Hierdurch wird eine den Anforderungen des Gelenkes an den natürlichen Bewegungsablauf in optimaler Weise angenäherte Kombination aus einer abschnittsweisen Rollbewegung sowie einer Gleitbewegung erreicht.

Bei einer Anwendung des künstlichen Gelenks als eine Endoprothese für ein menschliches Kniegelenk liegt die Schwenkachse der medialen Funktionsfläche des femoralen Gelenkteils in Relation zur Schwenkachse der lateralen Funktionsfläche des femoralen Gelenkteils im Bereich des Gelenks nach anterior, nach anterior kaudal oder nach anterior kranial versetzt. Weiterhin besitzt die mediale Funktionsfläche des femoralen Gelenkteils zwei Schwenkachsen.

Die Erfindung lässt verschiedene Ausführungsformen zu. Zur weiteren Verdeutschung ihres Grundprinzips ist eine davon in der Zeichnung dargestellt und wird nachfolgend beschrieben. Diese zeigt jeweils in einer Prinzipdarstellung in
- Fig.1: ein erfindungsgemäßes Gelenk in einer perspektivischen Darstellung;
- Fig.2: eine geschnittene Darstellung eines lateralen Gelenkteils des in Figur 1 gezeigten Gelenks in einer Schnittdarstellung;
- Fig.3: eine geschnittene Darstellung eines medialen Gelenkteils des in Figur 1 gezeigten Gelenks in einer Schnittdarstellung.

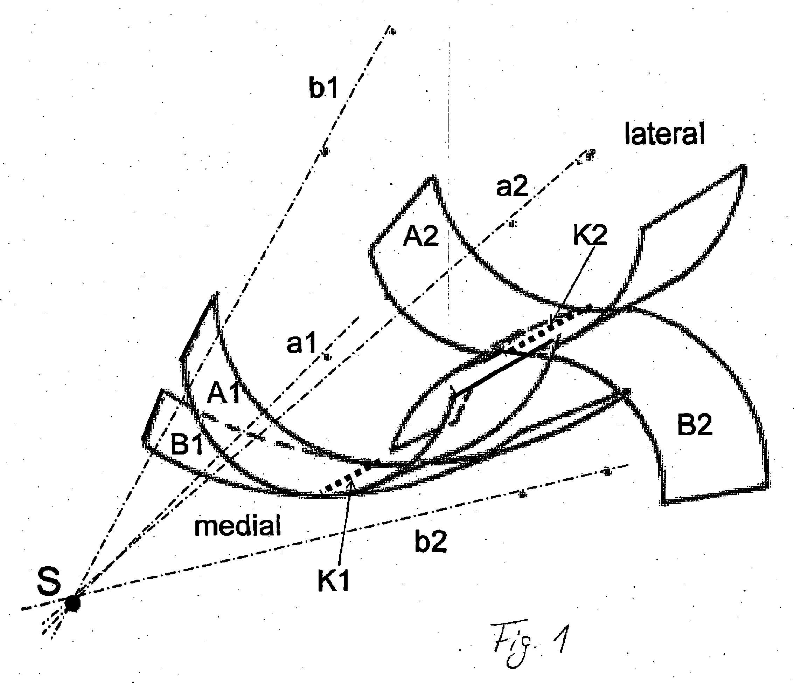

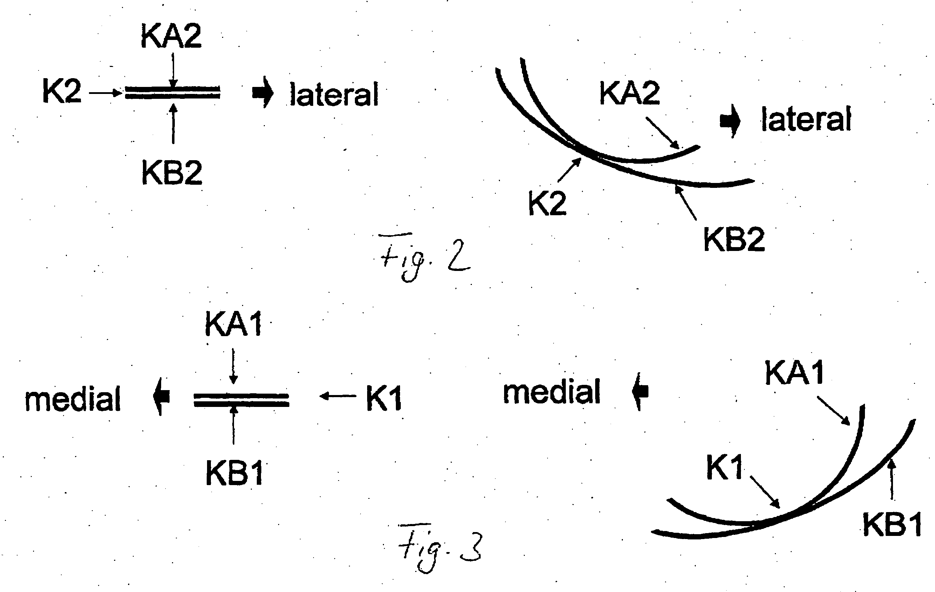

Ein erfindungsgemäßes künstliches Gelenk als Endoprothese für ein menschliches Gelenk wird anhand der Figuren 1 bis 3 näher dargestellt, die das Gelenk in einer perspektivische Darstellung sowie in den Figuren 2 und 3 in einer Schnittdarstellung eines lateralen Gelenkteils A und eines medialen Gelenkteils B zeigen. Jedes Gelenkteil A, B weist zwei miteinander in Wirkverbindung stehende, jeweils dem femoralen Gelenkteil einerseits und dem tibialen Gelenkteil andererseits zugeordnete sphäroidale Funktionsflächen A1, A2, B1, B2 auf. In der proximal-distalen Zuordnung sind die Funktionsflächen B1, B2 des medialen Gelenkteils B konkav-konvex und Funktionsflächen A1, A2 des lateralen Gelenkteils A konvex-konvex geformt. Die jeweiligen Schwenkachsen a1, a2, b1, b2 der Funktionsflächen A1, A2, B1, B2 jedes Gelenkteils A, B sind dabei zueinander geneigt und schneiden einander in einem gemeinsamen Schnittpunkt S. Der gemeinsame Schnittpunkt S der Schwenkachsen a1, a2, b1, b2 der Funktionsflächen A1, A2, B1. B2 liegt auf der medialen Seite außerhalb des Gelenks.

Der sich zwischen den Funktionsflächen A1, A2, B1, B2 ausbildende Kontakt K1, K2 wird anhand der Figuren 2 und 3 näher dargestellt, die eine entlang der Linie K1 und K2 geschnittene Ansicht der Funktionsflächen B1, B2 zeigen. Zu erkennen ist ein jeweiliger Kontaktbereich KA1, KB1 sowie KA2, KB2 als eine konvex-konkav Zuordnung der lediglich abschnittsweise gezeigten Funktionsflächen A1, A2, B1, B2. Der insbesondere punktförmige Kontakt K1, K2 entsteht dabei nicht in einem Tiefpunkt der Kontaktbereiche KB1 sowie KB2, sondern in einem dem der jeweils anderen Funktionsflächen A1, A2, B1. B2 desselben Gelenkteils A, B zugewandeten Bereich mittlerer Steigung.

## Patentansprüche

1. Künstliches Gelenk als Endoprothese für ein menschliches Gelenk, insbesondere Kniegelenk, bestehend aus mindestens zwei Gelenkteilen (A, B), wobei jedes Gelenkteil (A, B) zwei miteinander in Wirkverbindung stehende, jeweils dem ersten Gelenkteil (A) einerseits und dem zweiten Gelenkteil (B) andererseits zugeordnete Funktionsflächen (A1, A2, B1, B2) aufweist, wobei die beiden Funktionsflächen (A1, A2, B1, B2) jedes Gelenkteils (A, B) sphäroidal geformt und in der proximal-distalen Zuordnung konvex-konkav, konkav-konvex oder konvex-konvex geformt sind, und die Funktionsflächen (A1, A2, B1, B2) um jeweils eine Schwenkachse (a1, a2, b1, b2) schwenkbeweglich sind, wobei die beiden Schwenkachsen (a1, a2, b1, b2) der beiden Funktionsflächen (A1, A2, B1, B2) jedes Gelenkteils (A, B) zueinander geneigt sind, **dadurch gekennzeichnet, dass** die vier Schwenkachsen (a1, a2, b1, b2) der Funktionsflächen (A1, A2, B1, B2) der beiden Gelenkteile (A, B) sich einander in einem gemeinsamen Schnittpunkt (S) schneiden.

2. Künstliches Gelenk nach Anspruch 1, **dadurch gekennzeichnet, dass** die beiden Schwenkachsen (a1, a2, b1, b2) der Funktionsflächen (A1, A2, B1, B2) eines Gelenkteils (A, B) jeweils einen Schnittpunkt (S) aufweisen.

3. Künstliches Gelenk nach Anspruch 1 oder 2, **dadurch gekennzeichnet, dass** der gemeinsame Schnittpunkt (S) der Schwenkachsen (a1, a2, b1, b2) der Funktionsflächen (A1, A2, B1, B2) der beiden Gelenkteile (A, B) auf der medialen Seite liegt.

4. Künstliches Gelenk nach zumindest einem der vorhergehenden Ansprüche, **dadurch gekennzeichnet, dass** der gemeinsame Schnittpunkt (S) der Schwenkachsen (a1, a2, b1, b2) der Funktionsflächen (A1, A2, B1, B2) außerhalb der beiden Gelenkteile (A, B) liegt.

5. Künstliches Gelenk nach zumindest einem der vorhergehenden Ansprüche, **dadurch gekennzeichnet, dass** alle Schwenkachsen (a1, a2, b1, b2) der als Gelenkköpfe ausgeführtten der Funktionsflächen (A1, A2, B1, B2) eines Gelenkteils (A, B) übereinstimmen.

6. Künstliches Gelenk nach zumindest einem der vorhergehenden Ansprüche, **dadurch gekennzeichnet, dass** die jeweilige Funktionsfläche (A1, A2, B1, B2) zumindest eines Gelenkteils (A, B) aus zwei rotationssymmetrischen Körpern zusammengesetzt ist.

7. Künstliches Gelenk nach zumindest einem der vorhergehenden. Ansprüche, **dadurch gekennzeichnet, dass** die Funktionsflächen (A1, A2, B1, B2) in Bezug auf ihre jeweilige Schwenkachse (a1, a2, b1, b2) als ein Rotationskörper ausgeführt sind.

8. Künstliches Gelenk nach zumindest einem der vorhergehenden Ansprüche, **dadurch gekennzeichnet, dass** die Funktionsflächen (A1, A2, B1, B2) abschnittsweise kegelstumpfförmig ausgeführt sind.

9. Künstliches Gelenk nach zumindest einem der vorhergehenden Ansprüche, **dadurch gekennzeichnet, dass** die Funktionsflächen (A1, A2, B1, B2) in Abhängigkeit des Flexionswinkels des Gelenkes entweder aufeinander abrollen oder gleiten.

10. Künstliches Gelenk nach zumindest einem der vorhergehenden Ansprüche, **dadurch gekennzeichnet, dass** das Abroll-/Gleitverhältnis in Abhängigkeit des Flexionswinkels des Gelenkes veränderlich ist.

11. Künstliches Gelenk als Endoprothese für ein menschliches Kniegelenk nach zumindest einem der vorhergehenden Ansprüche, **dadurch gekennzeichnet, dass** die Gelenkteile (A, B) zum vollständigen oder teilweisen Ersatz des Femur und/oder der Tibia ausgeführt sind.

12. Künstliches Gelenk als Endoprothese für ein menschliches Kniegelenk nach zumindest einem der vorhergehenden Ansprüche, **dadurch gekennzeichnet, dass** die Schwenkachse (b1, b2) der medialen Funktionsfläche (B1, B2) des femoralen Gelenkteils (B) in Relation zur Schwenkachse (a1, a2) der lateralen Funktionsfläche (A1, A2) des femoralen Gelenkteils (A) im Bereich des Gelenks nach anterior, nach anterior kaudal oder nach anterior kranial versetzt liegt.

13. Künstliches Gelenk als Endoprothese für ein menschliches Kniegelenk nach zumindest einem der vorhergehenden Ansprüche, **dadurch gekennzeichnet, dass** die mediale Funktionsfläche des femoralen Gelenkteils zwei Schwenkachsen besitzt.

14. Künstliches Gelenk als Endoprothese für ein menschliches Kniegelenk nach zumindest einem der vorhergehenden Ansprüche, **dadurch gekennzeichnet, dass** alle Schwenkachsen des femoralen Gelenkteils zusammenfallen.

15. Künstliches Gelenk als Endoprothese für ein menschliches Kniegelenk nach zumindest einem der vorhergehenden Ansprüche, **dadurch gekennzeichnet, dass** die beiden Schwenkachsen des tibialen Gelenkteils zusammenfallen.

## Claims

1. Artificial joint as endoprosthesis for a human joint, in particular a knee joint, composed of at least two joint parts (A, B), each joint part (A, B) having two operatively connected function surfaces (A1, A2, B1, B2) assigned in each case on the one hand to the first joint part (A) and on the other hand to the second joint part (B), the two function surfaces (A1, A2, B1, B2) of each joint part (A, B) having a spheroidal shape and being convex-concave, concave-convex or convex-convex in the proximal-distal direction, and the function surfaces (A1, A2, B1, B2) each being pivotable about a pivot axis (a1, a2, b1, b2), the two pivot axes (a1, a2, b1, b2) of the two function surfaces (A1, A2, B1, B2) of each joint part (A, B) being inclined relative to each other, **characterized in that** the four pivot axes (a1, a2, b1, b2) of the function surfaces (A1, A2, B1, B2) of the two joint parts (A, B) intersect one another at a common intersection point (S).

2. Artificial joint according to Claim 1, **characterized in that** the two pivot axes (a1, a2, b1, b2) of the function surfaces (A1, A2, B1, B2) of a joint part (A, B) each have an intersection point (S).

3. Artificial joint according to Claim 1 or 2, **characterized in that** the common intersection point (S) of the pivot axes (a1, a2, b1, b2) of the function surfaces (A1, A2, B1, B2) of the two joint parts (A, B) lies on the medial side.

4. Artificial joint according to at least one of the preceding claims, **characterized in that** the common intersection point (S) of the pivot axes (a1, a2, b1, b2) of the function surfaces (A1, A2, B1, B2) lies outside the two joint parts (A, B).

5. Artificial joint according to at least one of the preceding claims, **characterized in that** all the pivot axes (a1, a2, b1, b2) of the function surfaces (A1, A2, B1, B2), designed as joint heads, of a joint part (A, B) coincide.

6. Artificial joint according to at least one of the preceding claims, **characterized in that** the respective function surface (A1, A2, B1, B2) of at least one joint part (A, B) is composed of two rotationally symmetrical bodies.

7. Artificial joint according to at least one of the preceding claims, **characterized in that** the function surfaces (A1, A2, B1, B2) are designed as a rotation body with respect to their respective pivot axis (a1, a2, b1, b2).

8. Artificial joint according to at least one of the preceding claims, **characterized in that** the function surfaces (A1, A2, B1, B2) are frustoconical in areas.

9. Artificial joint according to at least one of the preceding claims, **characterized in that** the function surfaces (A1, A2, B1, B2) either roll or slide on one another depending on the angle of flexion of the joint.

10. Artificial joint according to at least one of the preceding claims, **characterized in that** the rolling/sliding ratio can vary depending on the angle of flexion of the joint.

11. Artificial joint as endoprosthesis for a human knee joint according to at least one of the preceding claims, **characterized in that** the joint parts (A, B) are designed for total or partial replacement of the femur and/or tibia.

12. Artificial joint as endoprosthesis for a human knee joint according to at least one of the preceding claims, **characterized in that** the pivot axis (b1, b2) of the medial function surface (B1, B2) of the femoral joint part (B), seen in relation to the pivot axis (a1, a2) of the lateral function surface (A1, A2) of the femoral joint part (A), lies offset anteriorly, anteriorly and caudad, or anteriorly and craniad.

13. Artificial joint as endoprosthesis for a human knee joint according to at least one of the preceding claims, **characterized in that** the medial function surface of the femoral joint part has two pivot axes.

14. Artificial joint as endoprosthesis for a human knee joint according to at least one of the preceding claims, **characterized in that** all the pivot axes of the femoral joint part coincide.

15. Artificial joint as endoprosthesis for a human knee joint according to at least one of the preceding claims, **characterized in that** the two pivot axes of the tibial joint part coincide.

## Revendications

1. Articulation artificielle sous forme d'endoprothèse pour une articulation humaine, en particulier une articulation du genou, se composant d'au moins deux parties d'articulation (A, B), dans laquelle chaque partie d'articulation (A, B) comprend deux faces fonctionnelles (A1, A2, B1, B2) associées respectivement à la première partie d'articulation (A) d'une part et à la deuxième partie d'articulation (B) d'autre part et se trouvant en liaison active l'une avec l'autre, dans laquelle les deux faces fonctionnelles (A1, A2, B1, B2) de chaque partie d'articulation (A, B) ont une forme sphéroïdale et sont de forme convexe-concave, concave-convexe ou convexe-convexe dans leur association proximale-distale, et les faces fonctionnelles (A1, A2, B1, B2) sont mobiles en mouvement pivotant autour d'un axe de pivotement respectif (a1, a2, b1, b2), dans laquelle les deux axes de pivotement (a1, a2, b1, b2) des deux faces fonctionnelles (A1, A2, B1, B2) de chaque partie d'articulation (A, B) sont inclinées l'une par rapport à l'autre, **caractérisée en ce que** les quatre axes de pivotement (a1, a2, b1, b2) des faces fonctionnelles (A1, A2, B1, B2) des deux parties d'articulation (A, B) se coupent mutuellement en un point d'intersection commun (S).

2. Articulation artificielle selon la revendication 1, **caractérisée en ce que** les deux axes de pivotement (a1, a2, b1, b2) des faces fonctionnelles (A1, A2, B1, B2) d'une partie d'articulation (A, B) présentent chaque fois un point d'intersection (S).

3. Articulation artificielle selon la revendication 1 ou 2, **caractérisée en ce que** le point d'intersection commun (S) des axes de pivotement (a1, a2, b1, b2) des faces fonctionnelles (A1, A2, B1, B2) des deux parties d'articulation (A, B) se situe sur le côté médial.

4. Articulation artificielle selon au moins une des revendications précédentes, **caractérisée en ce que** le point d'intersection commun (S) des axes de pivotement (a1, a2, b1, b2) des faces fonctionnelles (A1, A2, B1, B2) se situe à l'extérieur des deux parties d'articulation (A, B).

5. Articulation artificielle selon au moins une des revendications précédentes, **caractérisée en ce que** tous les axes de pivotement (a1, a2, b1, b2) des faces fonctionnelles (A1, A2, B1, B2) réalisées en forme de tête d'articulation d'une partie d'articulation (A, B) coïncident.

6. Articulation artificielle selon au moins une des revendications précédentes, **caractérisée en ce que** la face fonctionnelle respective (A1, A2, B1, B2) d'au moins une partie d'articulation (A, B) est composée de deux corps à symétrie de révolution.

7. Articulation artificielle selon au moins une des revendications précédentes, **caractérisée en ce que** les faces fonctionnelles (A1, A2, B1, B2) sont réalisées en forme de corps de rotation par rapport à leurs axes de pivotement respectifs (a1, a2, b1, b2).

8. Articulation artificielle selon au moins une des revendications précédentes, **caractérisée en ce que** les faces fonctionnelles (A1, A2, B1, B2) sont réalisées par parties en forme de tronc de cône.

9. Articulation artificielle selon au moins une des revendications précédentes, **caractérisée en ce que** les faces fonctionnelles (A1, A2, B1, B2) roulent ou glissent l'une sur l'autre en fonction de l'angle de flexion de l'articulation.

10. Articulation artificielle selon au moins une des revendications précédentes, **caractérisée en ce que** le rapport de roulement/glissement est variable en fonction de l'angle de flexion de l'articulation.

11. Articulation artificielle sous forme d'endoprothèse pour une articulation humaine du genou selon au moins une des revendications précédentes, **caractérisée en ce que** les parties d'articulation (A, B) sont réalisées en vue du remplacement complet ou partiel du fémur et/ou du tibia.

12. Articulation artificielle sous forme d'endoprothèse pour une articulation humaine du genou selon au moins une des revendications précédentes, **caractérisée en ce que** l'axe de pivotement (b1, b2) de la face fonctionnelle médiale (B1, B2) de la partie d'articulation fémorale (B) est déplacée vers le côté antérieur, antéro-caudal ou antéro-cranien relativement à l'axe de pivotement (a1, a2) de la face fonctionnelle latérale (A1, A2) de la partie d'articulation fémorale (A) au nivaeu de l'articulation.

13. Articulation artificielle sous forme d'endoprothèse pour une articulation humaine du genou selon au moins une des revendications précédentes, **caractérisée en ce que** la face fonctionnelle médiale de la partie d'articulation fémorale présente deux axes de pivotement.

14. Articulation artificielle sous forme d'endoprothèse pour une articulation humaine du genou selon au moins une des revendications précédentes, **caractérisée en ce que** tous les axes de pivotement de la partie d'articulation fémorale coïncident.

15. Articulation artificielle sous forme d'endoprothèse pour une articulation humaine du genou selon au moins une des revendications précédentes, **caractérisée en ce que** les deux axes de pivotement de la partie d'articulation tibiale coïncident.
